# EUROPÄISCHE PATENTANMELDUNG

(11) **EP 2 574 591 A2**
(43) Veröffentlichungstag der Anmeldung: **03.04.2013**
(21) Anmeldenummer: 12181860.3
(22) Anmeldetag: 27.08.2012
(51) Int. Cl.: B67B 1/04

(54) **Verfahren zum Verschließen von Flaschen und Vorrichtung zum Bestrahlen von Korken in Flaschen**

(30) Priorität: 28.09.2011 DE 102011054007; 29.09.2011 DE 102011054039; 07.10.2011 DE 102011054306
(71) Anmelder: Brunner, Johann, 83666 Waarkirchen (DE)
(72) Erfinder: Brunner, Johann, 83666 Waarkirchen (DE)
(74) Vertreter: Viering, Jentschura & Partner

(57) **Zusammenfassung**

Bei einem Verfahren zum Vergrößern des Volumens von Korken wird dieser einer hochfrequenten Strahlung mit bestimmter Leistung für kurze Zeit ausgesetzt. Dabei erwärmt sich der Korken und vergrößert sein Volumen. Der gleiche Effekt tritt nicht nur bei Einzelkorken sondern auch bei Korkplatten oder Korkstreifen auf. Befindet sich der Korken bereits in einem Flaschenhals, so kann er sich nur begrenzt ausdehnen, wodurch Hohlräume und Risse in dem Korken verschlossen oder minimiert werden. Zudem werden bei hochfrequenter Bestrahlung von Kork schädliche Mikroorganismen abgetötet. Beide Effekte führen zu einer wesentlichen Verbesserung der Qualität von Korken und erlauben damit ein zuverlässigeres Verschließen von zum Beispiel Weinflaschen.

## Beschreibung

Der Erfindung zugrunde liegt die Erkenntnis des Erfinders, dass sich Kork bei Bestrahlung mit hochfrequenter Strahlung bei genügend Leistung ausdehnt.

Die Erfindung betrifft ein Verfahren zum Verschließen von Flaschen mit Korken und eine Vorrichtung zum Bestrahlen von Korken in Flaschen.

Wenn in dieser Patentschrift von hochfrequenter Strahlung bzw. von hochfrequenter Leistung geschrieben wird, so ist damit auch die Mikrowellenstrahlung einbezogen, die im üblichen Sprachgebrauch ab etwa 1 GHz beginnt.

Aus WO 2008/115086 A1 ist ein Verfahren zum Vergrößern des Volumens von Kork mittels Bestrahlung mit Mikrowellenstrahlung bekannt.

Der Kork für Korken wird aus der Rinde der Korkeiche gewonnen und ist ein teures Naturprodukt. Korkrinde wird zu Korkstreifen geschnitten, aus denen die Korken herausgestanzt werden. Anschließend werden die Korken verschiedenen bekannten Prozeduren unterzogen, auf die hier nicht näher eingegangen wird, damit sie zum Abdichten von Weinflaschen geeignet sind. Es können nur Korken verwendet werden, die bestimmte Qualitätskriterien erfüllen, wie zum Beispiel geringe Feuchtigkeit, kein Schimmelpilzbefall, keine zu großen Risse und Hohlräume, nur um einige zu nennen.

Es ist eine bekannte Tatsache, dass durch undichte oder verdorbene Korken den Winzern und der Weinindustrie jedes Jahr Schäden in Millionenhöhe entstehen. Bei den derzeit üblichen Verfahren zum Verschließen von Weinflaschen werden Korken durch eine mechanische Vorrichtung zusammengepresst und in die Flaschenhälse der Flaschen eingeführt. Problematisch ist, dass Korken Toleranzen in der Länge und in den Durchmessern sowie kleine Hohlräume und Risse aufweisen. Dies kann dazu führen, dass die Flaschen nicht richtig verschlossen werden und es zu Undichtigkeiten kommt.

Als Lösung dieses Problems wird ein Korken, der ganz oder teilweise in einem Flaschenhals steckt, mittels hochfrequenter Strahlung ganz oder teilweise bestrahlt, so dass er sich ausdehnt. Da dies in einem Flaschenhals nur sehr begrenzt möglich ist, verdichtet sich das Volumen des Korkens wodurch Hohlräume und Risse im Korken geschlossen oder minimiert werden. Dadurch wird die Qualität des Verschlusses mittels des Korkens gesteigert und die Flasche besser und sicherer abgedichtet. Durch dieses Verfahren lassen sich zukünftig Flaschen, insbesondere Weinflaschen sicherer und dauerhafter verschließen als es bislang möglich war.

Hochfrequente Strahlung hat die Eigenschaft, dass sie das Flaschenglas ohne große Dämpfungsverluste durchdringt. Bei der Bestrahlung durch hochfrequente Strahlung ist zudem nur ein eng begrenzter Teil des Flaschenhalses einschließlich des darin steckenden Korkens betroffen und nicht die restliche Flasche, wodurch eine unerwünschte Erwärmung der sich in der Flasche befindenden Flüssigkeit, beispielsweise von Wein, vermieden wird.

So konnte mit einem handelsüblichen Mikrowellenofen das Volumen eines Korkens, der bei 2,45 GHz für 10 Sekunden mit 600 Watt bestrahlt wurde, um mindestens 25 % dauerhaft vergrößert werden, ohne dass der Korken dabei überhitzt wurde, oder sonstige Schäden oder Einbußen an seiner Elastizität erkennbar waren.

Ein weiterer, aber bereits bekannter Vorteil der Bestrahlung von Korken durch hochfrequente Strahlung ist die Abtötung von schädlichen Mikrobakterien, die sich im Innern des Korkens befinden. Diese Mikroorganismen sind relativ häufig dafür verantwortlich, dass Korken stark geschädigt werden und der Wein, der sich in diesen Flaschen befindet, Qualitätseinbußen erleidet oder sogar ungenießbar wird.

Das Verfahren hat zudem noch den Vorteil, dass man die Ausdehnung des Korkens bis zu einem gewissen Grad bestimmen kann, weil man die Frequenz, sowie die Leistung und die Zeit der hochfrequenten Bestrahlung sehr genau regeln kann. Wie lange und bei welcher Leistung Korken in Flaschenhälsen bestrahlt werden sollen, um ein optimales Ergebnis zu erreichen, kann einfach empirisch, insbesondere durch Tests, beispielsweise abhängig vom Flaschenhalsdurchmesser und der Korkengröße ermittelt werden.

Mit hochfrequenter Strahlung kann man zum Beispiel auch nur eine Hälfte des Korkens vergrößern, während die andere Hälfte praktisch unverändert bleibt. Bei genau abgestimmter Positionierung der hochfrequenten Bestrahlung, die durch geeignete Anordnung von Elektroden zu erreichen ist, lässt sich sogar die Form des Korkens verändern. Damit wäre es zum Beispiel möglich, einen Naturkorken so zu bestrahlen, dass er eine ähnliche Form bekommt wie ein in Champagner Flaschen verwendeter Presskorken. Dies würde es der Schaumweinindustrie ermöglichen, zukünftig Naturkorken zum Abdichten der Schaumwein-Flaschen zu verwenden und nicht wie heute üblich Presskorken. Diese bestehen größtenteils aus zusammengepressten Korkabfällen und Korkstaub. Ein in der Form passender Naturkorken könnte ein weiteres Qualitätsmerkmal von Schaumweinen werden.

Beispielhaft werden in einigen Ausführungen Vorrichtungen gezeigt, die das vorher beschriebene Verfahren zum Erwärmen von Korken mittels hochfrequenter Strahlung in Flaschenhälsen ermöglichen würden. Diese Vorrichtungen sind nur beispielhaft und erheben keinen Anspruch auf technische Vollständigkeit.

Es zeigen:
- Figur 1: eine Seitenansicht einer Vorrichtung zum Bestrahlen von Korken in Flaschen gemäß verschiedenen Ausführungsbeispielen;
- Figur 2: eine weitere Seitenansicht der Vorrichtung gemäß Figur 1;
- Figur 3: eine Draufsicht der Vorrichtung gemäß Figur 1;
- Figur 4: eine weitere Draufsicht der Vorrichtung gemäß Figur 3;
- Figur 5: ein Blockschaltbild der Vorrichtung gemäß verschiedenen Ausführungsbeispielen;
- Figur 6: ein weiteres Blockschaltbild der Vorrichtung gemäß verschiedenen Ausführungsbeispielen;
- Figur 7: eine Vorrichtung zum Bestrahlen von Korken Flaschen gemäß verschiedenen Ausführungsbeispielen;
- Figur 8: eine Detailansicht der Vorrichtung gemäß Figur 7;
- Figur 9: ein Beispiel für einen Korken in einem Flaschenhals;
- Figur 10: ein Beispiel für einen Korken in einem Flaschenhals;
- Figur 11: ein Beispiel für einen Korken in einem Flaschenhals;
- Figur 12: ein Beispiel für einen Korken in einem Flaschenhals;
- Figur 13: eine Vorrichtung zum Bestrahlen von Korken in Flaschen gemäß verschiedenen Ausführungsbeispielen in einem ersten Betriebszustand;
- Figur 14: die Vorrichtung gemäß Figur 13 in einem zweiten Betriebszustand;
- Figur 15: die Vorrichtung gemäß Figur 13 in einem dritten Betriebszustand;
- Figur 16: eine Vorrichtung zum Bestrahlen von Korken in Flaschen gemäß verschiedenen Ausführungsbeispielen;
- Figur 17: eine Vorrichtung zum Bestrahlen von Korken in Flaschen gemäß verschiedenen Ausführungsbeispielen;
- Figur 18: ein Ablaufdiagramm eines Verfahrens zum Verschließen von Flaschen gemäß verschiedenen Ausführungsbeispielen.

In der folgenden ausführlichen Beschreibung wird auf die beigefügten Zeichnungen Bezug genommen, die Teil dieser bilden und in denen zur Veranschaulichung spezifische Ausführungsformen gezeigt sind, in denen die Erfindung ausgeübt werden kann. In dieser Hinsicht wird Richtungsterminologie wie etwa "oben", "unten", "vorne", "hinten", "vorderes", "hinteres", usw. mit Bezug auf die Orientierung der beschriebenen Figur(en) verwendet. Da Komponenten von Ausführungsformen in einer Anzahl verschiedener Orientierungen positioniert werden können, dient die Richtungsterminologie zur Veranschaulichung und ist auf keinerlei Weise einschränkend. Es versteht sich, dass die Merkmale der hierin beschriebenen verschiedenen beispielhaften Ausführungsformen miteinander kombiniert werden können, sofern nicht spezifisch anders angegeben. Die folgende ausführliche Beschreibung ist deshalb nicht in einschränkendem Sinne aufzufassen, und der Schutzumfang der vorliegenden Erfindung wird durch die angefügten Ansprüche definiert.

Im Rahmen dieser Beschreibung werden Begriffe wie "angeschlossen" sowie "eingekoppelt" verwendet zum Beschreiben sowohl eines direkten oder indirekten Anschlusses sowie einer direkten oder indirekten Kopplung. In den Figuren werden identische oder ähnliche Elemente mit identischen Bezugszeichen versehen, soweit dies zweckmäßig ist.

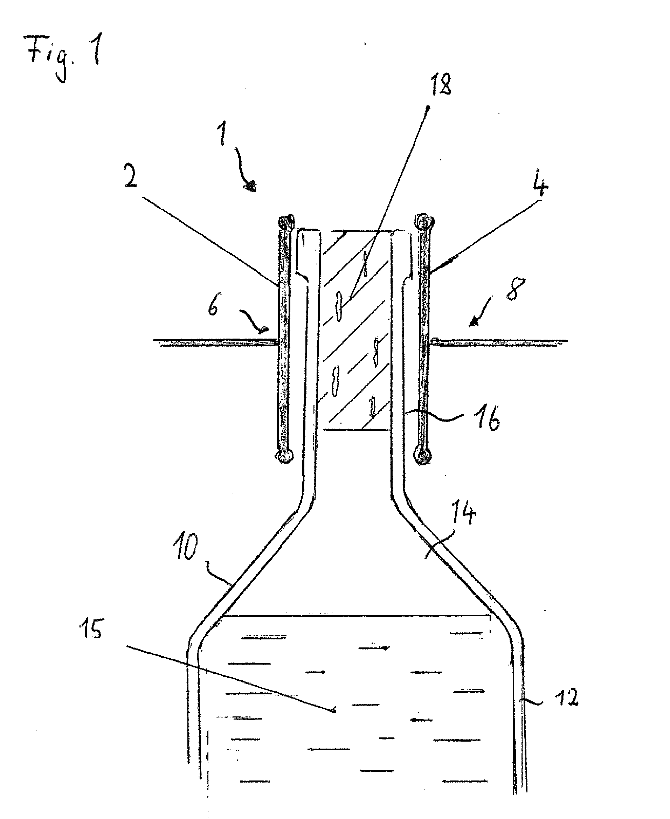

Figur 1 zeigt eine Vorrichtung 1 zum Bestrahlen von Korken in Flaschen 10 gemäß verschiedenen Ausführungsbeispielen. Die Flasche 10 hat eine Flaschenwand 12 und umschließt einen Innenraum 14, in dem eine Flüssigkeit 15, beispielsweise Wein, enthalten ist. In einem Flaschenhals 16 der Flasche 10 ist zum Verschließen der Flasche 10 ein Korken 18 angeordnet. Der Korken 10 ist ein Naturkorken, der aus der Rinde der Korkeiche hergestellt ist, und kann beispielsweise aus einem Stück Kork gebildet sein oder aus vielen kleinen Korkteilen zusammengepresst sein. Mit Hilfe der Vorrichtung 1 kann der Korken 18 mit hochfrequenter Strahlung bestrahlt werden, wodurch der Korken 18 sich erwärmt und ausdehnt. Die Ausdehnung des Korkens 18 in dem Flaschenhals 16 führt zu einem dichten Verschließen der Flasche 10. Bevorzugt ist die Flasche 10 dauerhaft verschlossen, vorzugsweise flüssigkeitsdicht. Mit hochfrequenter Strahlung ist beispielsweise Strahlung zwischen 10 KHz und 50 GHz gemeint

Eine erste Elektrode 2 und eine zweite Elektrode 4 sind um den Flaschenhals 16 herum angeordnet und umgeben den Flaschenhals 16 in radialer Richtung des Korkens 18 zumindest teilweise. An beide Elektroden 2, 4 wird ein um 180° phasenverschobenes Hochfrequenzsignal angelegt, das von einem Hochfrequenzgenerator erzeugt wird.

Die beiden Elektroden 2, 4 bilden zugleich einen Aufnahmebereich zum Aufnehmen des Flaschenhalses 16. Ferner können die beiden Elektroden in einem Gehäuse angeordnet sein, wobei das Gehäuse so ausgebildet sein kann, dass es eine Umgebung des Aufnahmebereichs und der Elektroden 2, 4 vor der Strahlung schützt. Die beiden Elektroden 2, 4 bilden weiter eine Anregungseinheit zum Erzeugen hochfrequenter Strahlung in dem Aufnahmebereich. Um eine möglichst gleichmäßige Ausdehnung des Korkens 18 zu erreichen, ist es zweckmäßig, wenn sich entweder die beiden Elektroden 2, 4 während der Bestrahlungszeit um die Flasche 10 drehen oder die Flasche 10 sich dreht.

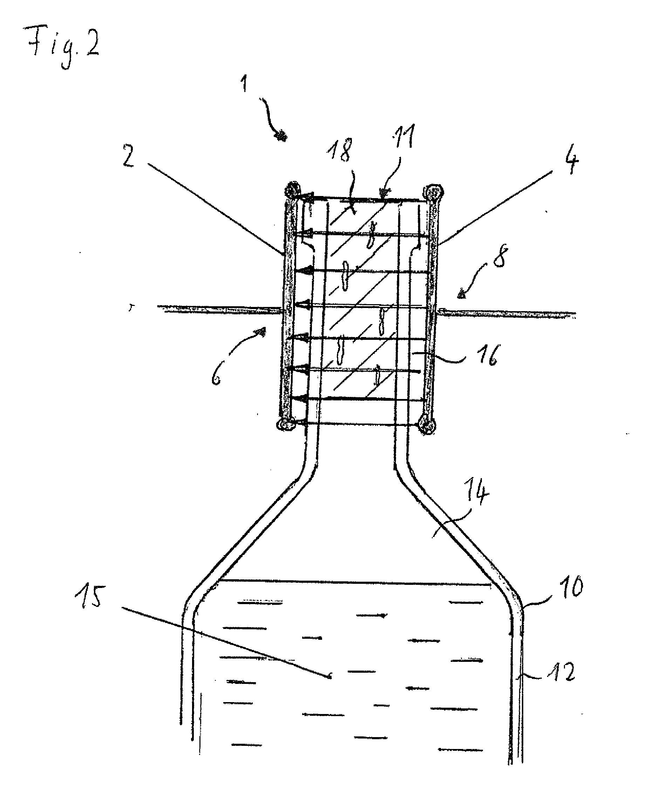

Figur 2 zeigt die Vorrichtung 1 gemäß Figur 1 mit einem zwischen den beiden Elektroden 2, 4 angelegten um 180° verschobenen Hochfrequenz-Wechselfeld 11.

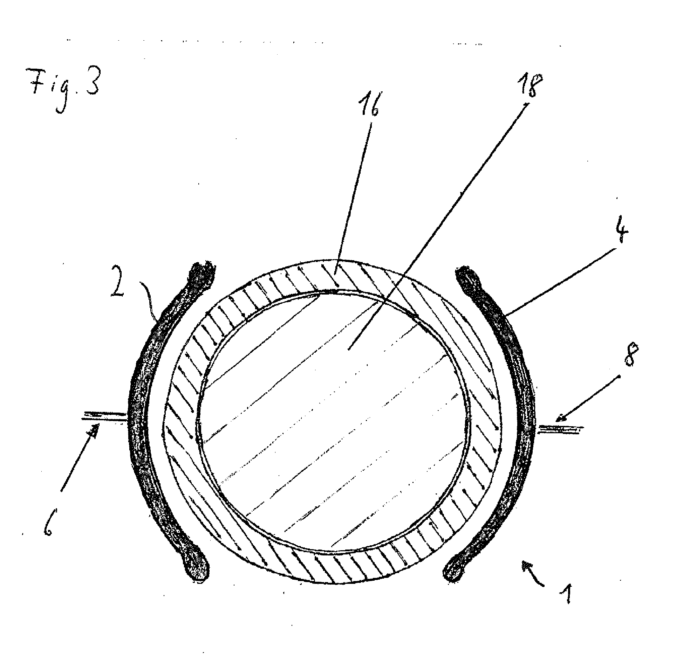

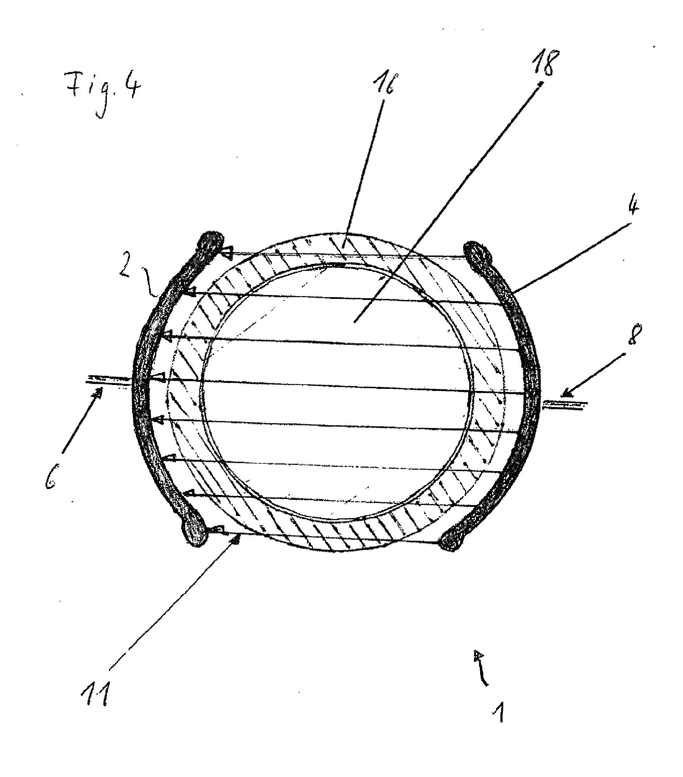

Figur 3 zeigt die Vorrichtung 1 gemäß Figur 1 in Draufsicht und Figur 4 zeigt die Vorrichtung 1 gemäß Figur 3 mit dem Hochfrequenz-Wechselfeld 11. Das Hochfrequenz-Wechselfeld 11 ist in den Figuren 2 und 4 durch mehrere parallele Pfeile dargestellt, wobei sich die Richtung des Feldes ständig um 180° ändert.

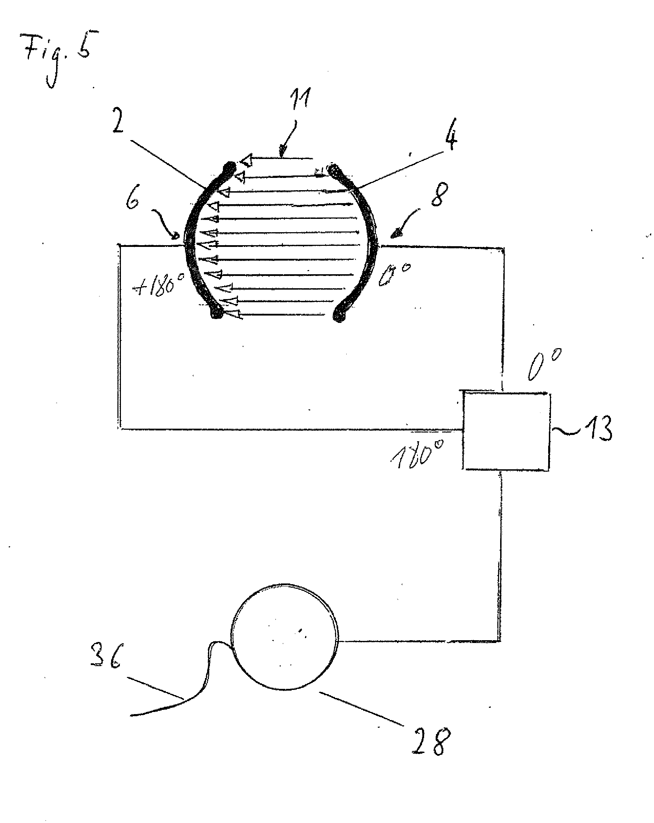

Figur 5 zeigt ein einfaches Blockschaltbild der in den Figuren 1 bis 4 gezeigten Vorrichtung 1 nach verschiedenen Ausführungsbeispielen. Die beiden Elektroden 2, 4 sind mit einem Leistungsteiler Phasenschieber 13 verbunden, welcher mit einem Hochfrequenz Leistungsgenerator 28 verbunden ist. Der Leistungsgenerator 28 wird über eine Stromversorgung 36 mit Strom versorgt.

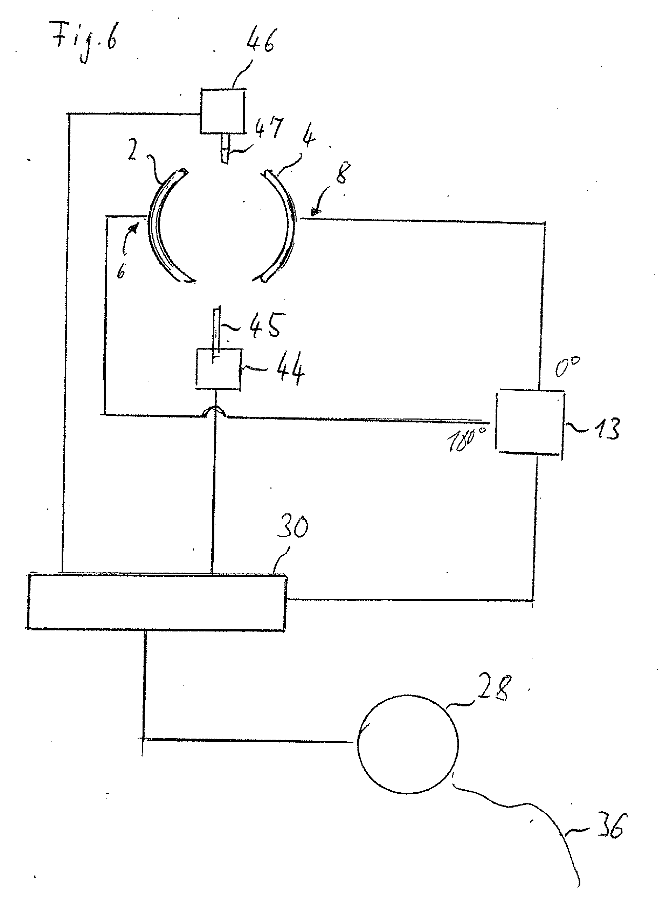

Figur 6 zeigt ein erweitertes Blockschaltbild der in den Figuren 1 bis 5 gezeigten Vorrichtung 1 nach verschiedenen Ausführungsbeispielen. Zusätzlich ist noch eine Steuereinheit 30 eingefügt. Sie dient dazu, den Hochfrequenz Leistungsgenerator 28 zu regeln. Der Leistungsdetektor 44 weist eine Messantenne 45 auf. Der Temperaturdetektor 46 weist einen Temperaturfühler 47 auf.

Die Funktionsweise der vorstehend erläuterten Vorrichtung 1 ist folgende: An den beiden Elektroden 2, 4 wird ein um 180° phasenverschobenes Hochfrequenzsignal angelegt, das von dem Leistungsgenerator 28 erzeugt wird.

Dadurch entsteht ein hochfrequentes elektrisches Wechselfeld zwischen beiden Elektroden 2, 4, das zum Beispiel bei einer Frequenz von 2,45 GHz, also 2,45 x 10⁹ mal in der Sekunde, um 180° die Richtung wechselt. Dadurch werden, wenn das Hochfrequenzsignal genügend Leistung aufweist und damit das elektrische Wechselfeld genügend stark ist, im Korken 18 Wassermoleküle durch Reibung erhitzt, was zu einer Erwärmung und zu einer Ausdehnung des Korkens 18 führt. Es ist jedoch nicht ausgeschlossen, dass und gegebenenfalls in wie weit im Korken 18 vorhandene Mikroorganismen dazu beitragen, dass sich der Korken 18 bei Bestrahlung ausdehnt.

Um die elektrischen Verluste möglichst gering zu halten und einen hohen Wirkungsgrad zu erzielen, sollten die Elektroden 2, 4 eine möglichst geringe Kapazität zu der Masse des Gehäuses aufweisen, in das die Elektroden 2, 4 gegebenenfalls eingebaut werden. Dies kann man z.B. dadurch erreichen, dass der Abstand zu der Masse des Gehäuses genau definiert wird in Abhängigkeit zur Wellenlänge der Strahlung. So sollte der Abstand zwischen den Elektroden 2, 4 und dem Gehäuse bei einer Frequenz von 2,45 GHz zwischen 0,5 cm und 1 cm sein. Die Kapazitätsverluste ließen sich auch minimieren, indem man zum Beispiel das Gehäuseinnere mit Teflon auskleidet. Zu beachten ist auch der Abstand beider Elektroden 2, 4 zueinander, wobei der Abstand in dem vorstehend gezeigten Ausführungsbeispiel durch den Durchmesser des von den Elektroden 2, 4 teilweise umschlossenen Kreises gegeben ist. Dieser Durchmesserabstand sollte zum Beispiel bei einer Frequenz von 2,45 GHz in etwa 3 cm betragen, was etwas weniger als einer viertelt Wellenlänge entspricht.

Mit Hilfe der Steuereinheit 30, die auch Controller genannt wird und den Detektoren 44, 46 lassen sich Ist-Parameter erfassen und auf Soll-Parameter nachregeln. So kann zum Beispiel die elektrische Leistung zwischen beiden Elektroden 2, 4 gemessen und mit einem vorgegeben Sollwert verglichen werden und falls nötig regelt dann das Steuergerät 28 die Leistung nach. Weiter kann mittels des Temperaturdetektors 46, beispielsweise einem Infrarotsensor, die Temperatur des Flaschenhalses 16 gemessen und mit einem entsprechenden Temperatur-Sollwert verglichen werden, um falls nötig die Leistung oder die Dauer der Bestrahlung zu ändern.

Alternativ zu der gleichmäßigen Ausdehnung mit Hilfe der hochfrequenten Strahlung kann eine ungleichmäßige Ausdehnung des Korkens 18 erzielt werden, beispielsweise durch entsprechendes Ausbilden der Elektroden 2, 4. Beispielsweise kann der Korken 18 mit Hilfe der hochfrequenten Strahlung nur in einem axialen Teilabschnitt erwärmt werden, so dass sich der Korken 18 im Wesentlichen nur in diesem axialen Teilabschnitt ausdehnt. Dadurch kann beispielsweise aus einem vollständig zylindrischen Korken 18 beispielsweise ein zumindest teilweise konischer oder kegelförmiger Korken 18 gebildet werden. Dies kann ermöglichen, einen aus einem Kork-Stück gebildeten Korken 18 zum Verschließen einer Champagner-Flasche zu verwenden. Beispielsweise kann der Korken 18 nur teilweise in den Flaschenhals 16 eingeführt werden, so dass ein oberer Abschnitt aus dem Flaschenhals 16 nach außen hervorsteht. Ein unterer Abschnitt des Korkens 18 ist dann in dem Flaschenhals der Champagner-Flasche angeordnet. Daraufhin kann ausschließlich der in dem Flaschenhals angeordnete Teil des Korkens 18 bestrahlt werden, wodurch sich dieser erwärmt und ausdehnt und die Champagner-Flasche dicht verschließt. Alternativ oder zusätzlich kann auch der obere Abschnitt des Korkens 18 bestrahlt und ausgedehnt werden, so dass der vergrößerte Korken 18 den Rand des Flaschenhalses 16 überlappt, was zur typischen Form eines Champagner-Flaschen Korken führt.

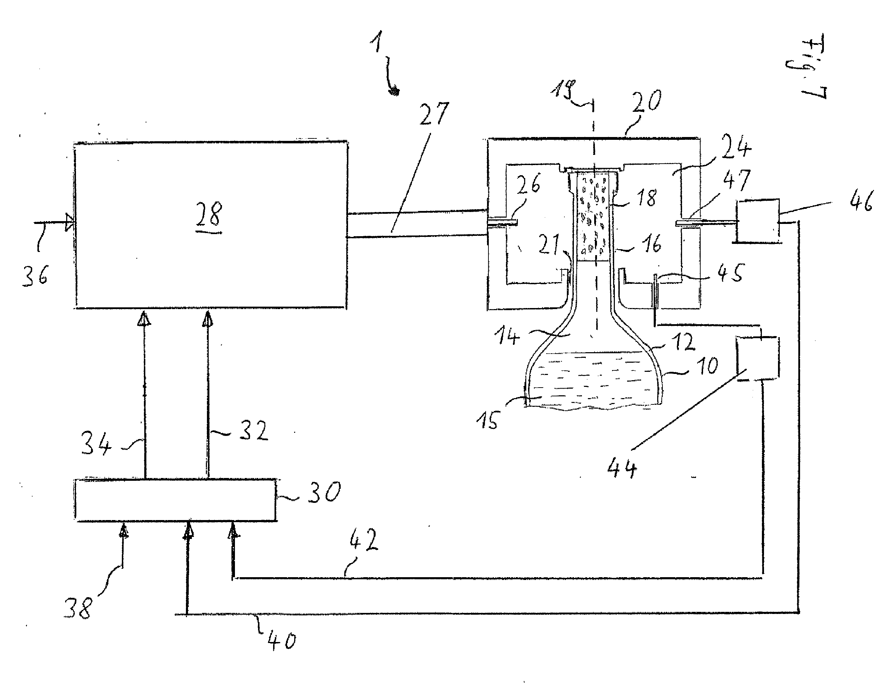

Figur 7 zeigt ein Blockschaltbild einer weiteren Vorrichtung 1 zum Bestrahlen von Korken 18 in Flaschen 10 gemäß verschiedenen Ausführungsbeispielen. Mit Hilfe der Vorrichtung 1 kann der Korken 18 mit hochfrequenter Strahlung, bestrahlt werden, wodurch sich der Korken 18 erwärmt und sich ausdehnt. Die Ausdehnung des Korkens 18 in dem Flaschenhals 16 führt zu einem dichten Verschließen der Flasche 10.

Der Flaschenhals 16 mit dem Korken 18 ragt durch eine Öffnung 21 in einen Hohlraumresonator hinein, so dass zumindest der Teil des Flaschenhalses 16 in radialer Richtung von dem Hohlraumresonator 20 umgeben ist, in dem der Korken 18 angeordnet ist. Der Hohlraumresonator 20, insbesondere der Hohlraum 24 des Hohlraumresonators 20 ist beispielsweise um eine Symmetrieachse 19 des Korkens 18 oder des Flaschenhalses 16 rotationssymmetrisch, also kreisrund, ausgebildet. Der Hohlraum 24 umfasst den Aufnahmebereich zum Aufnehmen des Flaschenhalses 16. In anderen Worten kann der Aufnahmebereich durch den Hohlraum 24 gebildet sein.

Ein Leistungsgenerator 28 und der Hohlraumresonator 20 sind über ein Koaxialkabel 27 miteinander verbunden. Über dieses Koaxialkabel 27 wird vom Leistungsgenerator 28 hochfrequente Leistung mittels einer Einkoppelantenne 26 in den Hohlraumresonator 20 eingespeist, die bei diesem Ausführungsbeispiel als Anregungseinheit dient. Als Verbindung zwischen Leistungsgenerator 28 und dem Hohlraumresonator 20 könnte auch ein Hohlleiter verwendet werden.

Die hochfrequente Strahlung kann beispielsweise in einem Frequenzbereich zwischen 10 KHz und 50 GHz, beispielsweise bei 860 MHz oder 2,45 GHz oder 5,8 GHz, um nur einige Frequenzen zu nennen, liegen. Der Leistungsgenerator 28 ist mit einer Steuereinheit 30 gekoppelt, über die eine Leistung 32 und eine Frequenz 34 der hochfrequenten Strahlung geregelt wird. Die Leistung 32 kann beispielsweise zwischen 100 W und 5000 W liegen, beispielsweise bei 450 W oder 600 W. Zum Erzeugen der hochfrequenten Strahlung ist der Leistungsgenerator 28 weiter mit einer Strom- bzw. Spannungsversorgung 36 gekoppelt. Der Steuereinheit 30 ist ein Leistungs-Sollwert 38 der hochfrequenten Strahlung vorgegeben. Alternativ dazu kann der Leistungs-Sollwert 38 über die Steuereinheit 30 vorgebbar sein.

Die Steuereinheit 30 ist zum Erfassen eines Leistungs-Istwerts 42 der hochfrequenten Strahlung in dem Hohlraum 24 mit einem Leistungsdetektor 44 und zum Erfassen einer Temperatur 40 des Flaschenhalses 16 mit einem Temperaturdetektor 46 verbunden. Der Leistungsdetektor 44 ist zum Erfassen der Strahlungsleistung in den Hohlraumresonator 20 mit einer in diesen ragenden Messantenne 45 verbunden. Der Temperaturdetektor 46 umfasst einen Temperaturfühler 47, wobei der Temperaturdetektor 46 und der Temperaturfühler 47 beispielsweise so ausgebildet sind, dass mit ihrer Hilfe von dem Korken 18 und/oder dem Flaschenhals 16 abgegebene Infrarotstrahlung erfassbar ist.

Durch die Steuereinheit 30, den Leistungsgenerator 28, die Einkoppelantenne 26 und den Hohlraumresonator 20 ist im Wesentlichen eine Vorrichtung 1 vorgegeben, mit deren Hilfe die hochfrequente Strahlung in dem Hohlraumresonator 20, insbesondere deren Leistung 32 und deren Frequenz 34 steuerbar ist. Mittels Leistungsdetektor 44, dessen Messantenne 45, und den Temperaturdetektor 46 und dessen Temperaturfühler 47 ist eine Regelung der Leistung der hochfrequenten Strahlung und damit der kontrollierten Ausdehnung des Korkens 18 möglicht. Beispielsweise kann die Steuereinheit 30 im Betrieb den Leistungs-Istwert 42 regelmäßig mit dem Leistungs-Sollwert 38 vergleichen und die Leistung 32 abhängig von dem Vergleich anpassen, so dass der Leistungs-Istwert 42 dem Leistungs-Sollwert 38 entspricht. Der Leistungs-Sollwert 38 kann beispielsweise fest vorgegeben sein oder von einem Anwender der Vorrichtung 1 vorgegeben werden. Die Temperatur 40 kann ebenfalls eine Regelgröße sein, die auf einen vorgegebenen oder einstellbaren Temperatur-Sollwert regelbar ist. Alternativ dazu kann die Temperatur 40 eine Kontroll- oder Sicherheitsgröße sein, durch die ein Sicherheitsgrenzwert vorgegeben ist, der nicht überschritten werden darf, wobei bei dessen Überschreitung eine Sicherheitsabschaltung der Vorrichtung 1 erfolgen kann. In anderen Worten kann eine maximale Temperatur der Erwärmung des Flaschenhalses 16 vorgegeben werden und falls die erfasste Temperatur 40 die maximale Temperatur erreicht oder überschreitet, kann der Leistungsgenerator 28 nachgeregelt werden. Die maximale Temperatur kann beispielsweise so vorgegeben werden, dass beim Bestrahlen des Korkens 18 das Glas der Flasche 10 nicht zerspringt.

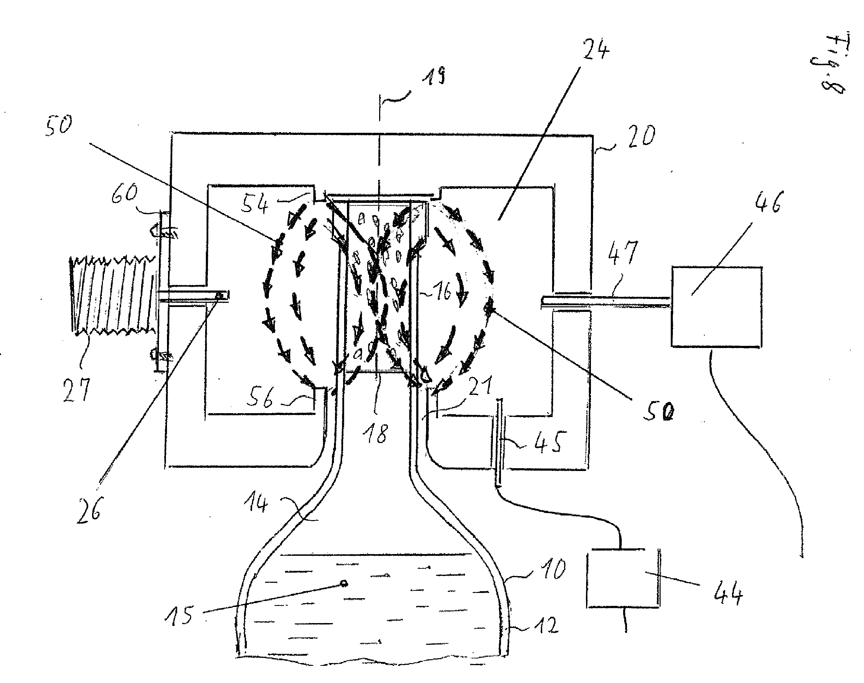

Figur 8 zeigt eine Detailansicht der Vorrichtung 1 zum Bestrahlen von Korken 18 in Flaschen 10 gemäß Figur 7 mit elektrischem Feld 50, das durch Pfeile dargestellt ist, insbesondere den Hohlraumresonator 20 mit dem darin aufgenommenen Flaschenhals 16. Der Hohlraumresonator 20 weist eine erste Kante 54 und eine zweite Kante 56 auf. Die beiden Kanten 54, 56 verlaufen kreisrund um den Flaschenhals 16 herum. Beim Betrieb der Vorrichtung 1 entsteht ein elektrisches Feld 50 in dem Hohlraum 24. Die Ausdehnung des elektrischen Feldes 50 wird durch den Abstand zwischen den beiden Kanten 54, 56 bestimmt. Darüber hinaus wirkt sich der Abstand zwischen den beiden Kanten 54, 56 auf die Eindringtiefe des elektrischen Feldes 50 in den Korken 18 aus, wobei bewirkt wird, dass sich der Korken 18 erwärmt und ausdehnt.

An einer Seite des Hohlraumresonators 20 ist eine Buchse 60 befestigt. Die Buchse 60 dient zum Verbinden des Koaxialkabels 27 mit der Einkoppelantenne 26 des Hohlraumresonators 20.

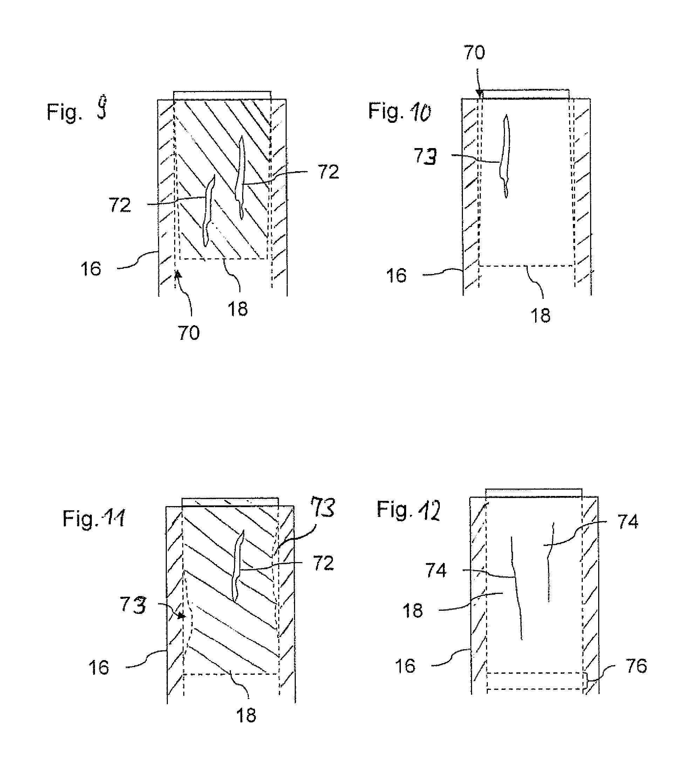

Figur 9 zeigt ein Beispiel für einen in den Flaschenhals 16 eingeführten Korken 18 vor der Behandlung in einer der Vorrichtungen 1 zum Bestrahlen von Korken 18 in Flaschen 10. Der Korken 18 kann beispielsweise in den Flaschenhals 16 eingeführt werden, indem der Korken 18 zunächst mechanisch zusammengepresst und dann in zusammengepresstem Zustand in den Flaschenhals 16 eingeführt wird, wo sich der Korken 18 wieder ausdehnt und den Flaschenhals 16 verschließt. Um eine möglichst gleichmäßige Ausdehnung des Korkens 18 zu erreichen, ist es zweckmäßig, wenn sich entweder die beiden Elektroden 2, 4 während der Bestrahlungszeit um die Flasche 10 drehen oder die Flasche 10 sich dreht.

Der Korken 18 kann Toleranzen und/oder Fehlbereiche aufweisen, aufgrund derer Flüssigkeit aus der Flasche 10 austreten kann. Dadurch kann sich die Qualität der Flüssigkeit 15 verschlechtern, beispielsweise kann Wein nach Kork schmecken oder verderben. Aufgrund der Toleranzen kann beispielsweise ein Spalt 70 zwischen dem Korken 18 und einer Innenwand des Flaschenhalses 16 entstehen. Die Fehlbereiche können beispielsweise aus Rissen oder Hohlräumen 72 bestehen.

Figur 10 zeigt ein weiteres Beispiel für einen in den Flaschenhals 16 eingeführten Korken 18 vor der Bestrahlung, wobei bei diesem Korken 18 der Spalt 70 an der Oberseite des Korkens 18 gebildet ist und wobei der Korken 18 zusätzlich eine Ausnehmung 73 an seiner Außenseite aufweist.

Figur 11 zeigt ein weiteres Beispiel für einen in den Flaschenhals 16 eingeführten Korken 18 vor der Behandlung in einer der Vorrichtungen 1 zum Bestrahlen von Korken 18 in Flaschen 10, wobei dieser Korken 18 Ausnehmungen 73 an seiner Außenseite und einen Hohlraum 72 aufweist.

Die Figuren 9, 10 und 11 zeigen somit einfache Beispiele für Korken 18, bei denen grundsätzlich die Gefahr besteht, dass sie die Flasche 10 nicht dauerhaft und/oder hermetisch abschließen, wodurch grundsätzlich die Gefahr besteht, dass die Flüssigkeit 15, insbesondere der Wein, bei der Lagerung Schaden nimmt.

Figur 12 zeigt ein Beispiel für einen in den Flaschenhals 16 eingeführten Korken 18 nach der Bestrahlung. Der Korken 18 liegt an seinem gesamten Außenumfang eng an der Innenwand des Flaschenhalses 16 an. Der Korken 18 weißt weder Hohlräume 72 noch Ausnehmungen 73 auf. Es sind zwar noch mit Hilfe der Vorrichtung 1 verschlossene Risse oder Hohlräume 74 erkennbar, diese sind jedoch derart zusammengepresst, dass keine Flüssigkeit aus der Flasche 10 austreten kann. In anderen Worten verschließt der Korken 18 die Flasche 10 bedeutend besser und zuverlässiger abgedichtet als dies bislang möglich war.

Bei der Behandlung des Korkens 18 mit Hilfe einer der Vorrichtungen 1 vergrößert sich das gesamte Volumen des Korkens 18. Der Korken 18 dehnt sich somit in alle Richtungen aus, insbesondere in radialer Richtung aber auch in axialer Richtung. Beispielsweise vergrößert sich der Korken in axialer Richtung um eine axiale Ausdehnung 76.

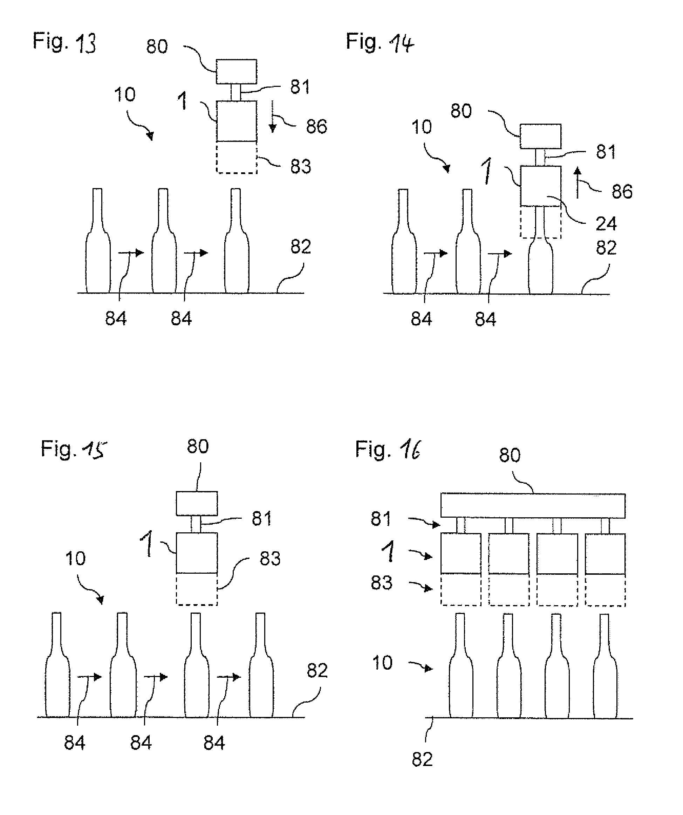

Figur 13 zeigt die Vorrichtung 1 zum Bestrahlen von Korken 18 in Flaschen 10 gemäß verschiedenen Ausführungsbeispielen in einem ersten Betriebszustand, wie sie beispielsweise zum Verschließen einer hohen Anzahl von Flaschen 10 ausgebildet sein kann. Die Vorrichtung 1 ist über einen Haltesteg 81 an einem Haltekörper 80 befestigt. Optional weist die Vorrichtung 1 einen Strahlenschutz 83 auf, der in einer einfachen Form als an der Vorrichtung 1 befestigter metallischer Zylinder ausgebildet sein kann. Auf einem Förderband 82 befinden sich mehrere Flaschen 10. Das Förderband 82 bewegt die Flaschen 10 in einer Förderrichtung 84. Die Vorrichtung 1 kann mit Hilfe des Haltekörpers 80 entlang einer Hubrichtung 86 bewegt werden. In dem ersten Betriebszustand ist die Vorrichtung 1 in einer Position, in der seine Unterkante und gegebenenfalls eine Unterkante des Strahlenschutzes 83 oberhalb des oberen Endes der Flaschen 10 angeordnet sind. Die Vorrichtung 1 wird in Hubrichtung 86 hin zu der Flasche 10 bewegt, die sich genau unter der Vorrichtung 1 befindet.

Figur 14 zeigt die Vorrichtung 1 zum Bestrahlen von Korken 18 in Flaschen 10 gemäß Figur 13 in einem zweiten Betriebszustand, in dem die Vorrichtung 1 so angeordnet ist, dass der Flaschenhals 16 der Flasche 10 mit dem darin angeordneten Korken 18 in dem Hohlraum 24 der Vorrichtung 1 angeordnet ist. Der zweite Betriebszustand folgt unmittelbar auf den ersten Betriebszustand. In dem zweiten Betriebszustand wird der Korken 18 mit Hilfe der hochfrequenten Strahlung bestrahlt.

Figur 15 zeigt die Vorrichtung 1 zum Bestrahlen von Korken 18 in Flaschen 10 gemäß Figur 13 in einem dritten Betriebszustand, der auf den zweiten Betriebszustand folgt. In dem dritten Betriebszustand wird die verschlossene Flasche 10 weiterbefördert und eine neue Flasche 10 mit einem unbehandelten Korken 18 in ihrem Flaschenhals 16 wird unter die Vorrichtung 1 bewegt.

In den Figuren 13, 14 und 15 sind die einzelnen Flaschen 10 voneinander beabstandet auf dem Fließband 82 angeordnet. Die Flaschen 10 können aber auch so auf dem Fließband 82 angeordnet sein, dass sie sich gegenseitig berühren. Alternativ zu der vorstehend gezeigten Ausführungsform kann auch die Vorrichtung 1 ortsfest verbleiben, wobei dann die Flaschen 10 in vertikaler Richtung in die Vorrichtung 1 hinein und wieder heraus bewegt werden können. Eine zusätzliche Drehung der Flasche 10 um ihre Längsachse ermöglicht eine gleichmäßigere Ausdehnung des Korkens 18.

Figur 16 zeigt eine Vorderansicht auf eine mögliche Ausführungsform des Ausführungsbeispiels gemäß Figur 13, bei der nebeneinander vier Vorrichtungen 1 angeordnet sind. Bei dieser Ausführungsform können gleichzeitig vier nebeneinander angeordnete Flaschen 10 mit hochfrequenter Strahlung bestrahlt werden und dicht verschlossen werden. Dahinter können weitere Flaschen 10 in Vierer-Reihen folgen.

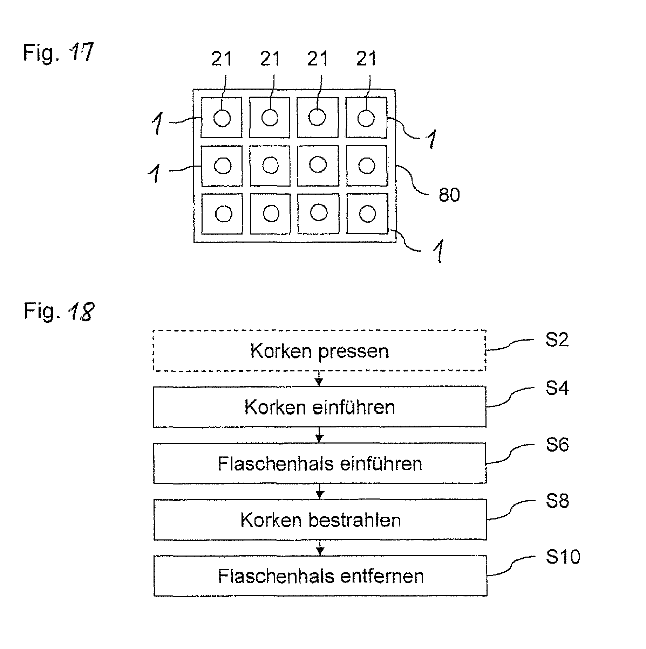

Figur 17 zeigt eine Vorrichtung 1 zum Bestrahlen von Korken 18 in Flaschen 10 gemäß verschiedenen Ausführungsbeispielen in einer Ansicht von unten, wobei gezeigt ist, dass mehrere Einzel-Vorrichtungen 1, beispielsweise zwölf, in Reihen und Spalten angeordnet sind. Dadurch kann eine Vielzahl von Flaschen 10, beispielsweise zwölf, gleichzeitig bestrahlt werden und damit dicht verschlossen werden.

Figur 18 zeigt ein Ablaufdiagramm eines Verfahrens zum Verschließen von Flaschen 10 gemäß verschiedenen Ausführungsbeispielen. Das Verfahren eignet sich dazu, eine oder mehrere Flaschen 10, beispielsweise Weinflaschen, nach dem Befüllen mit Hilfe je eines Korkens 18 dicht zu verschließen.

In einem Schritt S2, der optional ausgeführt werden kann, wird der Korken 18 derart in radialer Richtung zusammengepresst, dass sein äußerer Durchmesser nahezu gleich oder kleiner ist als ein innerer Durchmesser des Flaschenhalses 16. Alternativ dazu kann der äußere Durchmesser des Korkens 18 so gewählt werden, dass der Korken 18 ohne Zusammenpressen in den Flaschenhals 16 einführbar ist.

In einem Schritt S4 wird der Korken 18, gegebenenfalls der zusammengepresste Korken 18, in den Falschenhals 16 eingeführt. Falls der Korken 18 nicht zusammengepresst wird, kann beispielsweise der Durchmesser des Korkens 18 genau so groß gewählt werden, dass der Korken 18 leicht, beispielsweise ohne Zusammenpressen in den Flaschenhals 16 einführbar ist.

In einem Schritt S6 erfolgt eine Relativbewegung zwischen dem Flaschenhals 16 mit dem darin angeordneten Korken 18 und dem Aufnahmebereich, beispielsweise der Vorrichtung 1, wobei der Flaschenhals 16 beispielsweise durch Absenken der Vorrichtung 1 mit dem Aufnahmebereich oder durch Anheben der Flasche 10 in den Aufnahmebereich einführbar ist. Abhängig davon, ob der Korken 18 vollständig oder nur abschnittsweise bestrahlt werden soll, kann der in dem Flaschenhals 16 angeordnete Korken 18 vollständig oder teilweise in dem Aufnahmebereich angeordnet werden.

In einem Schritt S8 wird der Korken 18 mit der hochfrequenten Strahlung bestrahlt. Die zum dichten Verschließen der Flasche 10 benötigte Ausdehnung und Vergrößerung des Volumens des Korkens 18 kann innerhalb von wenigen Sekunden, bei größerer Leistung womöglich sogar noch schneller, erreicht werden. Beispielsweise kann der Korken 18 mit einer Leistung von 600 W und einer Frequenz von 2,45 GHz 10 Sekunden lang bestrahlt und erwärmt werden. Dies führt bei einem außerhalb der Flasche 10 angeordneten Korken 18 zu einer Volumenvergrößerung von mehr als 25 %. Ist der Korken 18 in dem Flaschenhals 16 angeordnet, so führt die Vergrößerung des Volumens des Korkens 18 zu dem dichteren Verschließen der Flasche 10. Das Glas der Flasche 10 wird von der hochfrequenten Strahlung ohne größere Verluste durchdrungen. Die Flüssigkeit 15, insbesondere der Wein, wird dabei nicht erwärmt und erleidet damit keine Qualitätseinbuße. Der Grad der Ausdehnung des Korkens 18 kann aufgrund der genauen Regelbarkeit der hochfrequenten Strahlung genau bestimmt werden. Auf diese Weise können auch unterschiedlich geformte Flaschen mit unterschiedlichen Ausmaßen besser als bisher dicht verschlossen werden.

In einem Schritt S10 wird der Flaschenhals 16 der dichten Flasche 10 aus dem Aufnahmebereich entfernt.

Das Vergrößern des Volumens von Korken 18 kann schon vor dem Einführen der Korken in Flaschen vorteilhaft genutzt werden, da gegenüber herkömmlichen Korken 18 nur noch Korken 18 mit geringeren Durchmessern benötigt werden. Der Durchmesser des Korkens 18 kann sogar kleiner als der Innendurchmesser der zu verschließenden Flasche 10 gewählt werden. So können aus einer vorgegebenen Menge an Kork-Rohstoff mehr Korken 18 als bisher gewonnen werden, wodurch Kosten gespart werden können. Die Korken 18 können dann vor dem Einführen in die Flaschen 10 mit Hilfe der hochfrequenten Strahlung vergrößert werden und dann beispielsweise mit Hilfe des herkömmlichen Pressverfahrens in die Flaschenhälse 16 eingeführt werden und die Flaschen 10 können so auf herkömmliche Weise mit den vergrößerten Korken 18 verschlossen werden und/oder die Flaschen 10 können zusätzlich oder alternativ mit Hilfe der vergrößerten Korken 18 und des vorstehend erläuterten Verfahrens verschlossen werden.

Möglich wäre zum Beispiel auch, dass die Vergrößerung des Korkens 18 in zwei Schritten erfolgt, sofern man nicht gleich im ersten Schritt die maximale Ausdehnung des Korkens 18 herbeiführt. Als erster Schritt wird der Korken 18 kurzeitig mit hochfrequenter Energie bestrahlt, so dass er sich erwärmt und sein Volumen bis zu einem vorgegeben Solldurchmesser vergrößert. Als zweiten Schritt wird der Korken 18 dann in den Flaschenhals 16 eingeführt und erneut bestrahlt, damit er sich nochmal ausdehnt und die Flasche 10 damit dicht verschlossen wird.

Ferner kann das Vergrößern von Kork im Allgemeinen unabhängig von dem Verschließen von Flaschen 10 vorteilhaft genutzt werden. Der natürliche Kork, der aus der Rinde der Korkeiche gewonnen wird, ist ein kostbarer und teurer Rohstoff. Egal für was dieser kostbare Rohstoff genutzt wird, beispielsweise als Bodenbelag oder als Wand- oder Deckenverkleidung, kann das Vergrößern seines Volumens dazu beitragen, aus einer bestimmten Menge Kork-Rohstoff, zum Beispiel Kork-Granulat, eine um mehr als 25 % größere Menge an Kork-Endprodukten zu erzeugen.

Die Erfindung ist nicht auf die angegebenen Ausführungsbeispiele beschränkt. Beispielsweise kann als Korken 18 ein Agglomerat-Korken verwendet werden, der aus vielen Korkstücken zusammengeklebt ist. Dabei können die Korkstücke vor dem Verkleben bzw. Verpressen und/oder nach dem Verkleben vergrößert werden. Ferner können mehr Elektroden 2, 4 vorgesehen sein und/oder die Elektroden 2, 4 können eine andere Form aufweisen. Ferner kann die Form der Vorrichtung 1 variieren und/oder das angegebene Verfahren zum Verschließen der Flaschen 10 kann zusätzliche oder alternativ Schritte aufweisen. Ferner kann die Flüssigkeit 15 auch Essig, Öl, Brandwein usw. umfassen.

### Bezugszeichenliste:

- 1: Vorrichtung
- 2: erste Elektrode
- 4: zweite Elektrode
- 6: erste Hochfrequenz-Einspeisung
- 8: zweite Hochfrequenz-Einspeisung
- 10: Flasche
- 12: Flaschenwand
- 14: Innenraum
- 15: Flüssigkeit
- 16: Flaschenhals
- 18: Korken
- 19: Symmetrieachse
- 20: Hohlraumresonator
- 21: Öffnung
- 24: Hohlraum
- 26: Einkoppelantenne
- 27: Koaxialkabel
- 28: Leistungsgenerator
- 30: Steuereinheit
- 32: Leistung
- 34: Frequenz
- 36: Stromversorgung
- 38: Leistungs-Sollwert
- 40: Temperatur
- 42: Leistungs-Istwert
- 44: Leistungsdetektor
- 45: Messantenne
- 46: Temperaturdetektor
- 47: Temperaturfühler
- 50: E-Feld
- 54: erste Kante
- 56: zweite Kante
- 60: Buchse
- 70: Spalt
- 72: Hohlraum oder Riss
- 73: Ausnehmung
- 74: verschlossener Hohlraum oder Riss
- 76: axiale Ausdehnung
- 80: Haltekörper
- 81: Haltesteg
- 82: Förderband
- 83: Strahlenschutz
- 84: Förderrichtung
- 86: Hubrichtung

- S2 - S10: Schritte zwei bis zehn

## Patentansprüche

1. Verfahren zum Verschließen von Flaschen (10), bei dem ein Korken (18), der ganz oder teilweise in einen Flaschenhals (16) eingeführt ist, mit hochfrequenter Strahlung durch das Glas hindurch ganz oder teilweise bestrahlt wird, so dass er sich ausdehnt und die Flasche (10) dicht verschlossen wird.

2. Verfahren nach Anspruch 1, bei dem die hochfrequente Strahlung eine Frequenz zwischen 10 KHz und 50 GHz hat.

3. Verfahren nach einem der vorstehenden Ansprüche, bei dem der Korken (18) oder der Kork mit einer Strahlungsleistung zwischen 100 W und 5000 W bestrahlt wird.

4. Verfahren nach einem der vorstehenden Ansprüche, bei dem der Korken (18) oder der Kork während einer Zeitdauer zwischen 0,1 s und 10 min bestrahlt wird.

5. Verfahren nach einem der vorstehenden Ansprüche, bei dem gleichzeitig mehrere Korken (18) in entsprechend mehreren Flaschen (10) bestrahlt werden.

6. Vorrichtung (1) zum Bestrahlen von Korken (18) in Flaschen (10), mit zumindest einem Aufnahmebereich, der so ausgebildet ist, dass darin ein in einem Flaschenhals (16) angeordneter Korken (18) aufgenommen werden kann, und zumindest einer Anregungseinheit zum Erzeugen hochfrequenter Strahlung in dem Aufnahmebereich zum ganzen oder teilweisen Bestrahlen des Korkens (18) in dem Flaschenhals (16) derart, dass sich der Korken (18) ausdehnt und den Flaschenhals (16) dicht abschließt.

7. Vorrichtung (1) nach Anspruch 6, bei der die Anregungseinheit zwei oder mehr Elektroden (2, 4) aufweist, an welche eine hochfrequente Leistung angelegt wird zum Erzeugen eines hochfrequenten Wechselfelds in dem Aufnahmebereich.

8. Vorrichtung (1) nach Anspruch 7, bei der die Elektroden (2, 4) zumindest teilweise den Aufnahmebereich begrenzen.

9. Vorrichtung (1) nach Anspruch 6, bei der ein Hohlraumresonator (20) angeordnet ist, der den Aufnahmebereich umfasst und der so ausgebildet ist, dass darin ein in einem Flaschenhals (16) angeordneter Korken (18) aufgenommen werden kann, und bei der als Anregungseinheit zum Erzeugen hochfrequenter Strahlung zumindest eine Einkoppelantenne (26) angeordnet ist, die zum Ausdehnen des Korkens (18) in dem Flaschenhals (16) die hochfrequente Strahlung in den Hohlraumresonator (20) einkoppelt, so dass der Korken (18) den Flaschenhals (16) dicht abschließt.

10. Vorrichtung (1) nach einem der Ansprüche 6 bis 9, mit einem Leistungsgenerator (28), der mit der Anregungseinheit elektrisch verbunden ist und der dazu geeignet ist, mit Hilfe der Anregungseinheit die hochfrequente Strahlung zu erzeugen.

11. Vorrichtung (1) nach einem der Ansprüche 6 bis 10, mit einem Temperatursensor (46), der so ausgebildet und angeordnet ist, dass mit seiner Hilfe die Temperatur des Korkens (18) ermittelt werden kann.

12. Vorrichtung (1) nach einem der Ansprüche 6 bis 11, mit einem Leistungsdetektor (44), der so ausgebildet und angeordnet ist, dass mit seiner Hilfe die Strahlungsleistung der hochfrequenten Strahlung ermittelt werden kann.

13. Vorrichtung (1) nach einem der Ansprüche 6 bis 12, mit einem Strahlenschutz, der so ausgebildet und angeordnet ist, dass er eine Umgebung des Aufnahmebereichs vor der hochfrequenten Strahlung schützt.

14. Vorrichtung (1) nach einem der Ansprüche 6 bis 13, die mehrere Aufnahmebereiche zum Aufnehmen entsprechender Flaschenhälse (16) zum Bestrahlen mit der hochfrequenten Strahlung aufweist.
